# EUROPÄISCHE PATENTANMELDUNG

(11) **EP 0 875 250 A1**
(43) Veröffentlichungstag der Anmeldung: **04.11.1998**
(21) Anmeldenummer: 97107064.4
(22) Anmeldetag: 29.04.1997
(51) Int. Cl.: A61K 35/78

(54) **Verwendung eines Extraktes aus Salvia officinalis bei der Bekämpfung der Dupuytren-Kontraktur**

(71) Anmelder: Heilmittelbetrieb Isernhagen GmbH, 61348 Bad Homburg (DE)
(72) Erfinder: Reinhard, Max, 61348 Bad Homburg (DE); Wolpert, Walter, 61381 Friedrichsdorf (DE)
(74) Vertreter: Schmidt, Horst, Dr.

(57) **Zusammenfassung**

Es wird die Verwendung eines Extraktes aus Salvia officinalis, insbesondere aus deren Blüten, bei der Bekämpfung der Dupuytren-Kontraktur beschrieben.

## Beschreibung

Die Erfindung betrifft die Verwendung eines Extraktes aus Salvia officinalis (Salbei) bei der Bekämpfung der Dupuytren-Kontraktur.

Die Dupuytren-Kontraktur ist eine aus unbekannter Ursache auftretende, fortschreitende narbige Schrumpfung und Verdickung der Beugekontraktur der zipfelartig ausgezogenen Sehnenplatte (Palmaraponeurose) der Hohlhand, wodurch es bei zunehmender Fingerverkrümmung, insbesondere des 4. und 5. Fingers, zu einer immer stärker werdenden Behinderung bei der Fingerstreckung kommt. Das Leiden, das Männer häufiger als Frauen befällt und ein- oder beidseitig auftreten kann, beginnt mit einer grübchenförmigen Einziehung in der Hohlhand, aus der sich allmählich, ganz schmerzlos Knötchen und Stränge bilden. Die Beugesehnen der betreffenden Finger selbst sind nicht erkrankt, sondern durch die Narbenstränge der Palmaraponeurose in ihrer Beweglichkeit beeinträchtigt.

Da die Erkrankung sich weder spontan zurückbildet noch eine konservative Behandlung, z.B. durch Massage, Wärmeeinwirkung und dergl., zu einem dauerhaften Erfolg führt, kann sie nur chirurgisch therapiert werden, wobei die gewucherten Schrumpfgewebe ausgeschnitten werden. Abgesehen davon, dass ein chirurgischer Eingriff immer mit Unannehmlichkeiten verbunden ist, hat es sich gezeigt, dass die dabei entstehenden Operationsnarben das später wiederkehrende Leiden noch verschlimmern können.

Aufgabe der Erfindung ist es, ein Arzneimittel zur Behandlung der Dupuytren-Kontraktur bereitzustellen.

Es wurde überraschenderweise festgestellt, dass sich aus Salbei (Salvia officinalis) ein Extrakt herstellen lässt, der sich zur Bekämpfung der Dupuytren-Kontraktur eignet. Gegenstand der Erfindung ist demzufolge die Verwendung eines Extraktes aus Salvia officinalis (Salbei) bei der Bekämpfung der Dupuytren-Kontraktur. Bevorzugter Extrakt für die Bahandlung dieser Krankheit ist eine Extrakt aus Blüten von Salvia officinalis.

Von der Salbeipflanze ist bisher die Verwendung von wässrigen Lösungen mit Inhaltsstoffen aus den Blättern gegen Schweissüberproduktion, Katarrhe sowie als Spül- und Gurgelmittel bekannt. Derartige Lösungen werden z.B. durch Behandlung von Salbeiblättern mit heissem Wasser gewonnen. Herkömmliche Verfahren zur Gewinnung von Salbeiextrakten umfassen neben der eigentlichen Extraktionsstufe, z.B. unter Verwendung von Alkohol, eine Stufe zum Abdestillieren des Extraktionsmittels bei Temperaturen über 100°C. Dabei werden viele Inhaltsstoffe thermisch geschädigt oder verändert.

Ferner ist aus DE-A-43 03 823 die Verwendung von Extrakten von Salbeiblüten zum Behandeln von Bluthochdruck, Durchblutungsstörungen und Wundheilungsstörungen bekannt.

Die Extraktion von Salvia officinalis kann mit Hilfe von beliebigen Extraktionsmitteln, wie Wasser, organischen Lösungsmitteln oder überkritischem CO₂, erfolgen. Ein Beispiel für ein organisches Lösungsmittel ist Ethanol. Wie bereits erwähnt, soll die Temperatur bei der Extraktion und bei einer sich gegebenenfalls daran anschliessenden Stufe zur mindestens teilweisen Entfernung des Extraktionsmittels, z.B. durch Destillation, bei 50°C oder darunter, vorzugsweise bei 40°C oder darunter, liegen, um eine thermische Beeinträchtigung der Inhaltsstoffe der Salbeipflanze zu verhindern. Dies bedeutet, dass bei der destillativen Trennung, der Druck soweit gesenkt werden muss, dass die genannte Temperaturobergrenze eingehalten werden kann.

Die Verwendung von überkritischem CO₂ zur Extraktion von Salbei wird besonders bevorzugt, da sie bei niedrigen Temperaturen durchgeführt werden kann und daher besonders schonend ist.

Die Extraktion mit überkritischem CO₂ kann in beliebigen, dafür geeigneten Vorrichtungen erfolgen. Aus den thermodynamischen Eigenschaften von CO₂, nämlich einer kritischen Temperatur von 31,3°C und einem kritischen Druck von 71,5 bar, ergeben sich die unteren Grenzen für die Temperatur und für den Druck bei der Extraktion.

Insbesondere ist es bei der CO₂-Extraktion bevorzugt, bei einer Temperatur von 40°C oder darunter zu arbeiten. Der Druck liegt vorzugsweise im Bereich von 90 bis 300 bar.

Die Extraktion kann solange fortgesetzt werden, bis alle mit überkritischem CO₂ extrahierbaren Inhaltsstoffe von Salvia officinalis extrahiert worden sind. Dies ist üblicherweise nach einer Dauer des Extraktionsverfahrens von 1 bis 2 Stunden der Fall. Möglich ist es jedoch auch, nur einen Teil der Inhaltsstoffe von Salvia officinalis zu extrahieren.

Ein Vorteil der Verwendung von überkritischem CO₂ als Extraktionsmittel im Vergleich zur Verwendung anderer Extraktionsmittel, wie Ethanol oder Wasser, besteht darin, dass bei Temperaturen unter 40°C extrahiert werden kann, während z.B. bei einer herkömmlichen alkoholischen Extraktion das Abdestillieren des Ethanols bei Temperaturen von über 100°C erforderlich ist.

Ein weiterer Vorteil der Extraktion mit überkritischem CO₂ besteht darin, dass lösungsmittelfreie Extrakte erhalten werden können. Eine ungünstige Beeinflussung der Heilwirkung des Extraktes durch Lösungsmittel, wie Ethanol, kann daher vermieden werden.

Der erfindungsgemässe verwendete Extrakt wird vorzugsweise aus Blüten von Salvia officinalis gewonnen. Die Blüten werden nach der Ernte vorzugsweise getrocknet, und zwar bei einer Temperatur von 40°C oder darunter. Die Trocknungstemperatur wird wie die Extraktionstemperatur vergleichsweise niedrig gewählt, um eine schonende Behandlung der Salbeiblüten zu erlauben. Auch der Einsatz von tiefgefrorenen Blüten kommt in Frage.

Der Extrakt aus Blüten von Salvia officinalis wird in pastenartiger Konsistenz erhalten. Dieser Extrakt kann als wirksamer Bestandteil des Arzneimittels verwendet werden. Das Arzneimittel wird vorzugsweise in Form eines Injektionspräparats bereitgestellt. Es ist keine Trägerflüssigkeit erforderlich, da der verwendete Salbeiextrakt bei Temperaturen von 30 bis 40°C bereits flüssig ist. Vorzugsweise wird dem Präparat ein Lokalanästhetikum zugesetzt, so dass das Einspritzen des Präparats gleichzeitig eine örtliche Betäubung bewirkt. Es kommen beliebige übliche Lokalanästhetika im Verhältnis von 1:1 zum Extrakt in Frage.

Zur Behandlung wird das Injektionspräparat direkt in den Bereich der betroffenen Sehne gespritzt, wo die Hauptspannung war. Durch die Injektion entsteht im verkürzten Sehnenbereich eine Entzündung. Das damit verbundene Ödem macht das Sehnengewebe auf natürliche Weise dehnfähig. Durch Quengelung in dieser Phase lässt sich eine Verlängerung der Sehne erreichen, was eine Besserung der Fingerfunktion ergibt. Die Behandlung wird vorzugsweise im Abstand von einer oder mehreren Wochen mehrmals wiederholt.

Nachstehend wird die Erfindung durch Beispiele näher erläutert.

### Beispiel 1

Das Beispiel betrifft die Herstellung eines CO₂-Gesamtextraktes aus Blüten von Salvia officinalis. Dazu wurden handgepflückte, getrocknete Blüten 2 Stunden bei einem Druck von 300 bar (vollständige Extraktion) und einer Temperatur von 40°C mit CO₂ extrahiert. Aus 15,2 kg Salbeiblüten wurden dabei 623 g Extrakt gewonnen. Dies entspricht einer Extraktausbeute von 4,1 %. Der Extrakt fiel als Paste an.

### Beispiel 2

Das Beispiel betrifft die Gewinnung eines CO₂-Selektivextraktes aus Blüten von Salvia officinalis. Die Extraktionsbedingungen umfassten eine Extraktionsszeit von 2 Stunden, einen Druck von 90 bar (selektive Extraktion) und eine Temperatur von 40°C. Aus 1,7 kg eingesetzten getrockneten Blüten wurden dabei 14 g Extrakt gewonnen. Dies entspricht einer Extraktausbeute von 0,8 %. Der Extrakt fiel als Paste an.

### Beispiel 3 (Behandlungsbeispiel)

Ein männlicher Patient (Alter 60 Jahre), der am vierten und fünften Finger der linken Hand an der Dupuytren-Kontraktur litt, erhielt eine Injektion des Präparats gemäss Beispiel 2 (Dosierung 0,2 ml pro Finger) in den Bereich der betroffenen Sehnen. Durch die Injektion kam es zu einer Ödembildung. Durch Quengelung konnte eine Verlängerung der Sehnen erreicht werden. Diese Behandlung wurde insgesamt 3 mal (mit Behandlungspausen von jeweils mehreren Wochen) durchgeführt.

Es wurde folgende Besserung erreicht:

### Ausgangszustand:

- Beugekontraktur: am Ringfinger: 30°
am kleinen Finger: 90°

Nach dreimaliger Injektion von jeweils 0,2 ml Präparat ergaben sich nach ingesamt vier Wochen folgende Werte:
- Ringfinger aktiv:: 5°
- kleiner Finger aktiv:: 30°
- Ringfinger passiv:: 0°
- kleiner Finger passiv:: 10°

"aktiv" bedeutet: Der Finger kann ohne Krafteinwirkung hochgestellt werden.
"passiv" bedeutet: Der Finger kann mit Krafteinwirkung (beispielsweise mit der jeweils anderen Hand) möglichst weit zurückgebogen werden.

Im Rahmen der Erfindung können auch andere Pflanzenteile als Blüten von Salvia officinalis, wie Wurzeln, Blätter oder Stengel, für die Extraktion herangezogen werden. Die Extraktion kann analog zur beschriebenen Extraktion der Blüten erfolgen.

## Patentansprüche

1. Verwendung eines Extraktes aus Salvia officinalis bei der Bekämpfung der Dupuytren-Kontraktur.

2. Verwendung gemäss Anspruch 1, dadurch gekennzeichnet, dass ein Extrakt aus Blüten von Salvia officinalis verwendet wird.
